Europäisches Patentamt

European Patent Office

Office européen des brevets

Veröffentlichungsnummer: **0 282 958**

**A1**

# EUROPÄISCHE PATENTANMELDUNG

Anmeldenummer: **88104041.4**

Anmeldetag: **15.03.88**

Int. Cl.⁴ **B01J 4/02** , B65D 88/66 ,
//A61M15/00

Priorität: **19.03.87 DE 3708933**

Veröffentlichungstag der Anmeldung:
**21.09.88 Patentblatt 88/38**

Benannte Vertragsstaaten:
**ES GR**

Anmelder: **BOEHRINGER INGELHEIM
ZENTRALE GMBH
Postfach 200
D-6507 Ingelheim am Rhein(DE)**

Anmelder: **Reents, Heinrich, Prof. Dr. Ing.
Magnolienweg 23
D-4750 Unna(DE)**

Erfinder: **Reents, Heinrich, Prof. Dr.-Ing.
Magnolienweg 23
D-4750 Unna(DE)**

Verfahren und Vorrichtung zur Dosierung von Pulvern.

Ein Verfahren zur Ausbringung feinster Pulver durch enge Öffnungen oder Düsen benutzt die Einwirkung von Schwingungen auf die Pulverteilchen, wobei die Schwingungsfrequenz bei der Frequenz der Eigenresonanz der Teilchen oder in der Nähe davon liegt. Eine Vorrichtung zur Durchführung des Verfahrens besteht im wesentlichen aus einem Vorratsbehälter (1), der mit einem Schwingungserzeuger (4, 5) gekoppelt und mit einer Düse (2) versehen ist.

FIG.1.

EP 0 282 958 A1

## Verfahren und Vorichtung zur Dosierung von Pulvern

Die Erfindung betrifft ein neues Verfahren zur Dosierung feinteiliger Feststoffe mittels mechanischer Schwingungen und Vorrichtungen zur Durchführung des Verfahrens.

Feste Stoffe haben eine rauhe Oberfläche. Dies bedingt hohe Reibbeiwerte, die bei Pulvern, z.B. feinstteiligen anorganischen Substanzen oder mikronisierten Arzneistoffen (bei denen außerdem van der Waals'schen Kräfte eine große Rolle spielen) den Austritt aus kleinen Öffnungen (Düsen) erschweren oder verhindern. Die Dosierung feiner und feinster fester Stoffe ist deshalb vielfach nur möglich, wenn diese Stoffe in Verbindung mit einem Trägermedium aus dem Vorratsbehälter herausgetrieben werden. Im Falle der Keramik-und Schmuckindustrie erfolgt dies, indem die Keramikteilchen mit Wasser vermischt und mit einem Pinsel oder durch Aufsprühen der Suspension aufgetragen werden. Mikronisierte Arzneistoffe werden meist mit Hilfe von Treibgasen in Aerosole, beispielsweise für die Inhalation oder die nasale Applikation, übergeführt.

Die erwähnten Ausbringungsverfahren sind jedoch nur dann wirksam, wenn eine homogene Mischung von Flußmittel/verflüssigtem Treibgas und suspendiertem Stoff vorliegt. Da in der Regel die feste und die flüssige Phase unterschiedliche Dichte haben, kommt es häufig, z.T. schon nach kurzer Standzeit, zu einer Entmischung. Als Folge davon ist es nach dem derzeitigen Stand der Technik in der Schmuckindustrie nicht möglich, die hochfeinen Werkstoffe vollautomatisch aufzutragen. Bei der Verwendung von Dosieraerosolen zu therapeutischen Zwecken muß vor der Applikation durch Schütteln für eine Homogenisierung der Suspension gesorgt werden. Die an sich unerwünschte Ausbringung von Treibgasen ist aber weiterhin notwendig.

Es ist schon versucht worden, die Rauhigkeit der Teilchen durch eine Vorbehandlung zu vermindern. Befriedigende Ergebnisse wurden damit jedoch nicht erzielt.

Besondere Schwierigkeiten stellen sich ein, wenn eine veränderbare Dosierung erforderlich ist. So kann es notwendig sein, daß bei einem Prozeß zunächst kleine Mengen eines Pulvers durch eine Düse durchfließen und erst in Abhängigkeit von den Prozeßparametern die Dosierleistung erhöht werden muß. Die bekannten Verfahren und Vorrichtungen vermögen dies nicht zu leisten.

Für die inhalative Anwendung von pharmazeutischen Zubereitungen kommt auch in Betracht, den Wirkstoff in Einzeldosen der eingeatmeten Luft beizumischen. Hierzu ist jedoch erforderlich, die einzelnen Dosen, z.B. in Hargelatinekapseln abzufüllen, wozu technisch aufwendige Verfahren benutzt werden müssen.

Aufgabe der Erfindung ist es, ein Verfahren und Vorrichtungen zu seiner Durchführung vorzuschlagen, womit es möglich ist, feste Wirkstoffe/Wirkstoffe sehr geringer Teilchengröße in gewünschtenfalls veränderbarer Dosierung aus einer Dosiervorrichtung in bestimmten Mengen abzugeben, ohne daß Treibgase oder Flußmittel verwendet werden.

Die Lösung dieser Aufgabe wird erfindungsgemäß dadurch erreicht, daß die auszubringenden Teilchen in einem mit einer kleinen Öffnung bzw. einer Düse versehenen Behälter so stark in Schwingung versetzt werden, daß sie durch die Öffnung/Düse herausfallen, solange der Schwingungsvorgang andauert.

Die erfindungsgemäße Lösung erfüllt zugleich folgende Anforderungen:

a) die ausgebrachte Menge pro Zeiteinheit kann konstant gehalten werden;

b) die ausgebrachte Menge kann den Prozeßanforderungen entsprechend variiert werden (unterschiedliche Ausbringungsmenge pro Zeiteinheit);

c) die Vorrichtung ist gut handhabbar;

c1) sie kann mit einfachen Einrichtungen in die Benutzungsstellung gebracht, benutzt und wieder entfernt werden. (In der Schmuckindustrie, wo häufig mehrere Farben (z.B. Emailpulver) nacheinander angewendet werden müssen, bietet die Koppelung entsprechend vieler Dosiereinheiten gemäß der Erfindung mit einer Positioniereinrichtung eine vorteilhafte Lösung.);

c2) im Falle der Medizintechnik kann die Vorrichtung so kompakt hergestellt werden, daß sie vom Benutzer gut mitgeführt werden kann;

d) die Vorrichtung kann mit einem an sich bekannten Verschlußmechanismus und gegebenenfalls mit einer Meßvorrichtung versehen werden, so daß die Ausbringungsmenge insgesamt genau definierbar ist;

e) das System ist preiswert herzustellen und umweltfreundlich.

Der Gegenstand der Erfindung kann überall angewendet werden, wo bestimmte, insbesondere geringe Mengen feiner Pulver abgemessen werden sollen. Geeignete Einsatzgebiete sind dementsprechend beispielsweise die Schmuck-und Keramikindustrie sowie die Medizintechnik. In dieser spielt die Verabreichung von Arzneimitteln in Pulverform durch Inhalation und auch nasale Applikation eine bedeutende Rolle. Bei Benutzung der Erfindung können nun - unter Vermeidung von Treibgasen - wie bei den Aerosolzubereitungen mehrfach genau

eine Halbschale immer befüllt werden, und aus der anderen kann der Werkstoff/Wirkstoff entnommen werden.

In einer weiteren Ausführungsform sind zwei oder mehrere Halbschalen oder auch nur eine in einem Schieber integriert und werden intermittierend unter die Düse geschoben.

In einer weiteren Ausführungsform ist das Dosiersystem Teil einer Handhabungseinrichtung. So ist vorgesehen, daß im Anwendungsfall Keramikindustrie ein Roboter die einzelnen Dosiersysteme mit jeweils eigenen Farben aus einer Vorrichtung entnimmt, sie zu dem Werkstück führt, ein Freigabesignal an die Steuerungs-/Regelungsschaltung des Dosiersystems gibt, die Keramikteilchen zusammen mit der Bewegung des Roboterarmes aufgetragen werden, und das Dosiersystem wieder abgelegt wird. In dieser Position wird es durch einen oberen Schließmechanismus aus einem Vorratsbehälter wieder automatisch befüllt.

In Rahmen der Medizintechnik sind ähnliche Anwendungen möglich. So kann z.B. das Dosiersystem derart kompakt sein, daß es in der Größenordnung einer kleinen Spraydose aufgebaut wird.

Die Ausbringung, insbesondere von extrem feinteiligen Pulvern (etwa mikronisierten Arzneistoffen) kann verbessert werden, indem man gröbere Teilchen (Größe > 10 $\mu$m) oder auch wesentlich kleinere Teilchen (Größe < 1 $\mu$m) des Arzneistoffs oder eines physiologisch annehmbaren Hilfsstoffs zumischt. Die Verbesserung der Fließfähigkeit mikronisierter Arzneistoffe und der Handhabbarkeit durch diese Maßnahme ist an sich bekannt.

Feuchtigkeitseinfluß auf den Inhalt der Düse oder des Vorratsbehälters können stören. Um den Feuchtigkeitseinfluß gering zu halten, kann z.B. im Vorratsbehälter ein durch eine geeignete Membran, z.B. Goretex® abgetrennter Raum vorgesehen werden, der ein Trocknungsmittel wie Silicagel aufnimmt. Insbesondere im Zusammenhang mit der Inhalationstherapie kann zwischen der Düse und dem Mundstück, durch das der Patient inhaliert, ein Einwegventil eingeschaltet werden. Damit läßt sich verhindern, daß beim Ausatmen die feuchte Luft an oder in die Düse gelangt. Auf jeden Fall ist es bei der Anwendung der Erfindung auf therapeutischem Gebiet ratsam, einen gut abdichtenden Verschluß der Düse vorzusehen, der nur bei der Entnahme des Pulvers geöffnet ist.

Ausführungsformen der Erfindung werden durch die beigefügten Figuren näher erläutert.

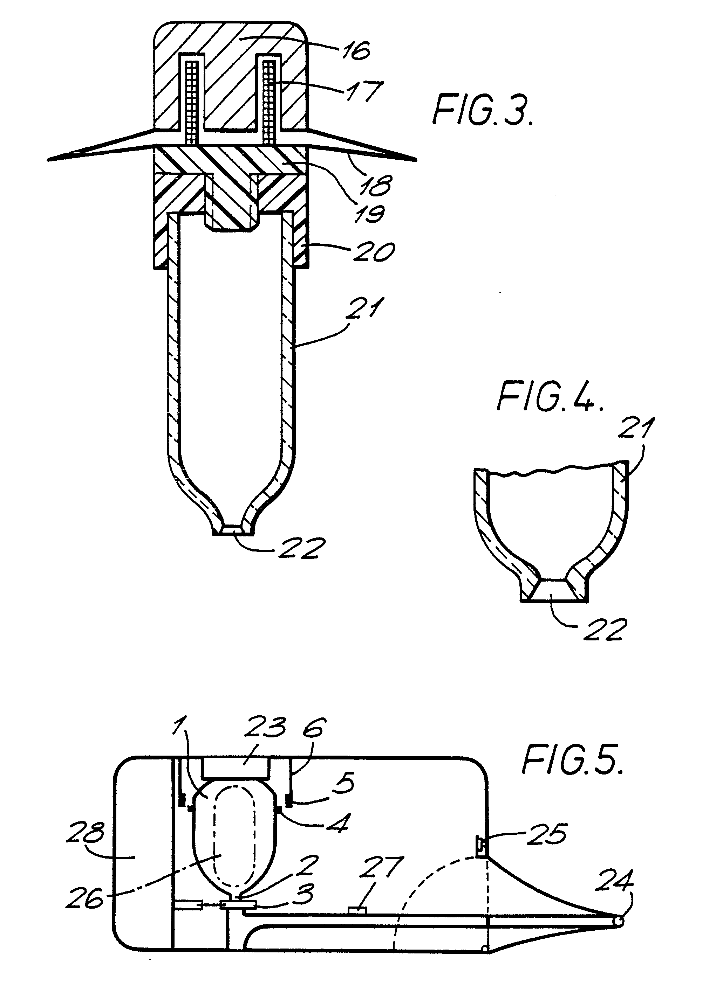

Figur 1 zeigt ein erfindungsgemäße Dosiersystem als einzelne Baugruppe. Die an seinem Vorratsbehälter 1 angesetzte Düse 2 kann durch einen elektromagnetisch betätigten Schieber 3 geöffnet bzw. verschlossen werden. Das schwingungserzeugende System von Spulen umfaßt die Sekundärwicklung 4 und die Primärwicklung 5, die innnerhalb der Haltevorrichtung 6 liegen. Fließt ein mit einer bestimmten Frequenz pulsierender Strom durch die Spule 5, so wird das System in Schwingung versetzt. Die Teilchen 7 lösen sich voneinander und fallen durch die Düsenöffnung.

Das Dosiersystem ist mit Hilfe eines flexiblen Schlauchs 8 mit einem größeren Vorratsbehälter verbunden. Der Zugang zu diesem Vorratsbehälter wird gesteuert über einen Schließer/Öffner 9. Der Schließer/Öffner ist wiederum mit Füllstandssensoren 10 und 11 gekoppelt. Dabei mißt der Sensor 10 den maximalen. der Sensor 11 den minimalen Füllstand.

Falls die Düse selbst nicht bewegt werden darf. z.B. im Falle exakter Positionierung, können die - schwingungserzeugenden Systeme in dem Vorratsbehälter integriert sein. Alternativ ist möglich, daß der Werkstoff einfach vermischt wird mit Partikeln, die durch elektromagnetische Felder in Bewegung versetzt werden können, z.B. kleine nicht rostende Eisenpartikel. Diese Partikel müssen jedoch so dimensioniert sein, daß sie nicht durch die Düsenöffnung fallen können.

Figur 2 zeigt die exakte Dosierung der Austrittsmenge mit Hilfe einer Meßvorrichtung. Die Düse ist durch 12 dargestellt. Die Meßvorrichtung ist in Form einer rotierenden Kreisscheibe 13 mit Vertiefungen 14 ausgeführt. In dem gezeigten Ausführungsbeispiel sind die Vertiefungen einfach durchgebohrte Löcher. Unterhalb der Löcher befindet sich im Düsenbereich eine feste Platte 15. Der Ablauf ist damit wie folgt: Im ersten Schritt wird die Meßvorrichtung 14 gefüllt. Anschließend wird die Kreisscheibe weitergedreht und die Teilchen fallen nach unten durch die Meßvorrichtung. Gleichzeitig wird die zweite Meßvorrichtung wieder gefüllt, so daß mit jedem Zyklus die abgemessene Menge Werkstoff/Wirkstoff herausfällt.

Figur 3 zeigt eine kompakte einfache Ausführungsform der Erfindung. Sie besteht im wesentlichen aus einem kleinen Lautsprecher mit dem Magneten 16, der Spule 17 und der Membran 18, auf die der Kunststoffzapfen 19 aufgeklebt ist. Er überträgt die Schwingungen des Lautsprechers auf den Inhalt der mit einer Kunststoffhülse 20 versehenen Glasdüse 21, die das auszubringende Pulver enthält. Die Öffnung 22 wird zweckmäßig mit einem an sich bekannten Verschluß versehen.

Mit Hilfe des Lautsprechers werden die elektrischen Schwingungen, die von einem Rechteckgenerator erzeugt werden, in mechanische Schwingungen umgewandelt. Der Lautsprecher, der hier verwandt wird, gehört zu den dynamischen Lautsprechern. Die Spule wird durch die Membran freischwingend im ringförmigen Luftspalt des Dauermagneten gehalten. Auf der Membran ist die Düse

dosierte Mengen Arzneipulver verabreicht werden.

Die erfindungsgemäße Vorichtung besteht im wesentlichen aus einer Ausbringungsdüse (gegebenenfalls verbunden mit einem Vorratsbehälter), gekoppelt mit einem mechanische Schwingungen erzeugenden System.

Die Koppelung kann mittelbar oder unmittelbar erfolgen. Im Falle der unmittelbaren Kopplung wird die Düse (gegebenenfalls mit Vorratsbehälter) direkt mit einer Spule verbunden. Ein durch die Spule fließender intermittierender Strom sorgt dafür, daß die Teilchen in dem Behälter in Schwingungen geraten. Im Bereich der Resonanz ist die Schwingung derart groß, daß sich die Teilchen nur noch wenig oder kaum berühren. Durch diesen Effekt ist die Rauhigkeit der Oberfläche keine prozeßbestimmende Größe mehr und auch die van der Waals'schen Kräfte werden überwunden. Durch die Eigenbewegung der Teile, hervorgerufen durch die Schwingung, kommt es nicht mehr zur Torbildung vor der Düsenöffnung; d.h. die Teilchen behindern sich nicht gegenseitig, sondern fallen in der gewünschten Menge durch den Düsenhals bzw. die Düsenöffnung.

Durch die Änderung der Frequenz und der Amplitude der zugeführten Energie kann die berührende Oberfläche exakt eingestellt werden. Ist die Frequenz weit von der Eigenresonanz der Teilchen entfernt, werden sich die Teilchen berühren - die durchfallende Teilchenmenge nimmt ab.

Da die Teilchengröße sich im allgemeinen in einem definierten Toleranzfeld befindet, kann man durch Änderung der Parameter Frequenz, Amplitude und Lage die Durchflußmenge exakt dosieren.

In einer anderen Ausführungsform der mittelbaren Kopplung, wird die Düse mit einer Membran verklebt. Die Membran ist gekoppelt mit einem - schwingungserzeugenden System, z.B. bestehend aus einer Spule und einem Oszillator. Derartige Systeme sind in Miniatur-oder Subminiaturausführung realisierbar, so daß den Anforderungen einer kompakten Ausführung Rechnung getragen wird.

Die Oszillatorfrequenz ist im obigen Beispiel veränderbar durch die Schwingkreisparameter (Widerstand, Induktivität, Kapazität), die Amplitude ist veränderbar durch die Regelung/Steuerung der Verstärkung.

Versuche mit gefertigten Mustern im Labor haben die exakte Dosierbarkeit bestätigt.

Koppelt man die Oszillatorschaltung mit Sensoren, so ist die Ausbringungsmenge veränderbar entsprechend den Prozeßanforderungen. So kann die ausfließende Menge von Wirkstoffen in medizinischen Prozessen dem Einatmungsstrom des Patienten angepaßt werden.

Um die Dosiergenauigkeit zu erhöhen, ist in einer weiteren Ausführungsform vorgesehen, daß der Füllstand des Dosiersystems laufend erfaßt wird und innerhalb vorgegebener Toleranzgrenzen geregelt wird. Dies kann z.B. durch den Einsatz optoelektronischer Sensoren oder durch der Einsatz von Gewichtssensoren geschehen.

In einer weiteren Ausführungsform werden die Schwingungen nicht elektromagnetisch in Form einer Spule mit Anker aufgebracht. sondern elektromotorisch. Das Dosiersystem wird gekoppelt mit einem Elektromotor mit Unwucht. Die Änderung der Frequenz und der Amplitude werden bewirkt durch die Änderung der Drehzahl des Motors und Veränderung seiner Unwucht.

In einer weiteren Ausführungsform wird die Schwingung durch mechanische Baugruppen aufgebracht, z.B. einen Federmotor mit Unwucht. Der Vorteil dieser Lösung ist darin zu sehen, daß eine externe Hilfsenergie in Form elektrischer Energie nicht zugeführt werden muß. Umweltprobleme durch Batterien sind somit ebenfalls vermeidbar.

In einer anderen Ausführungsform wird die Schwingung aufgebracht durch Einsatz der Pneumatik und/oder Fluidik.

In einer anderen Ausführungsform werden die Schwingungen aufgebracht durch Schwingungserzeuger, die auf dem Piezoeffekt basieren oder alternativ durch Ultraschall-Schwingungserzeuger.

In einer weiteren Ausführungsform, dies gilt insbesondere bei größeren Einheiten, werden die - schwingungserzeugenden Systeme direkt im Vorratsbehälter installiert. Dabei kann die Erregung von außen erfolgen durch elektromagnetische Felder oder durch direkte Energiezuführung in den Vorratsbehälter, gekoppelt mit der Düse.

Befindet sich das System nach der Dosierung in Ruhe, kann gegebenenfalls auf einen zusätzlichen Verschlußmechanismus verzichtet werden. Befindet sich das System jedoch in laufender Bewegung, ist ein Verschlußmechanismus unumgänglich. Dieser kann ausgeführt sein z.B. in Form eines Schiebers oder in Form einer sich verändernden Düsenöffnung. So ist in einer Ausführungsform vorgesehen, die Düsenöffnung elastisch zu gestalten. Sie ist damit sowohl mit Hilfe rein mechanischer als auch elektrischer oder elektromagnetischer Stellglieder leicht veränderbar.

In einer weiteren Ausführungsform wird die Düsenöffnung mit einer Meßvorrichtung gekoppelt. Die Meßvorrichtung gestaltet die Dosierung kleinster absoluter Mengen. Exakte Dosierungen von Wirkstoffen sind in der Medizintechnik unumgänglich.

Der Entnahmevorgang kann in den Schritten 1. Dosierung, 2. Entnahme ablaufen. In einer Ausführungsform sind z.B. zwei Meßvorrichtungen in Form von Halbschalen oder durchgebohrten Löchern mit Auffangvorrichtung vorgesehen. Die Halbschalen sind drehbar gelagert. Dadurch kann

befestigt, so daß die Schwingungen auf die Düse übertragen werden, die in axialer Richtung - schwingt. Durch geeignete Einstellung des Rechteckgenerators wird eine solche Frequenz erzeugt, die zur Resonanz in dem Pulver führt. Insbesondere für die Ausbringung mikronisierter Pulver kann die Düsenöffnung sehr klein sein (unter 1 mm Durchmesser).

Figur 4 zeigt eine Ausgestaltung der Düse 21, durch die die engste Stelle möglichst geringe axiale Ausdehnung hat, was durch eine nach außen konisch erweiterte Öffnung 22 erreicht wird.

In Figur 5 ist schematisch dargestellt, wie eine erfindungsgemäße Vorrichtung (im wesentlichen entsprechend Figur 1) in ein Gerät für die Inhalation pulverförmiger Arzneistoffe integriert werden kann. Das Vorratsgefäß 1 ist mit einer Düse 2 versehen, die durch den Schieber 3 geöffnet bzw. geschlossen werden kann. Zur Schwingungserzeugung dienen die Spulen 4, 5 mit der Halterung 6. Das Gerät ist mit einer Nachfüllvorrichtung 23, einem herausklappbaren Mundstück 24 mit dem Einschaltkontakt 25 und einem Sichtfenster 26 für die optische Füllstandskontrolle versehen. Als weitere Bauteile sind ein Unterdrucksensor und eine Elektronik 28 vorgesehen, wobei letztere Schwingungserzeugung und Regelung der einzelnen Operationen übernimmt.

Zur Benutzung wird zunächst das Mundstück 24 herausgeklappt, wobei der Einschaltkontakt 25 betätigt wird, wodurch für eine gewisse Zeitspanne die Elektronik scharf gemacht wird. Der Unterdrucksensor 27 erfaßt den Beginn der Einatmung und löst dann die Öffnung des Schiebers 3 aus. Die Elektronik 28 erzeugt nun Schwingungen, durch die eine gewünschte Dosis des Pulvers ausgebracht wird. Der Unterdrucksensor erfaßt dann das Ende der Einatmung und schaltet die Elektronik ab.

Alternativ kann beispielsweise auch eine kegelförmige Abdichtung für die Düse verwendet werden, die mittels elektronischer Steuerung für die Dauer der Schwingungserzeugung geöffnet wird.

Wie die Beispiele erkennen lassen, läßt sich die erfindungsgemäße Konzeption vom Fachmann ohne weiteres in vielfältiger Weise modifizieren.

Beispielsweise können auch mehrere Düsen vorgesehen sein oder die Düsenöffnung kann siebartig gestaltet werden.

**Ansprüche**

1. Verfahren zum dosierten Ausbringen von feinen Pulvern, dadurch gekennzeichnet, daß die Pulverteilchen durch mittelbares oder unmittelbares Aufbringen noch mechanischen Schwingungen auf die Teilchen durch eine Düse getrieben werden, wobei die Frequenz der Schwingungen vorzugsweise im Bereich der Eigenresonanz der Teilchen gewählt wird.

2. Verfahren nach Anspruch 1, dadurch gekennzeichnet, daß die Schwingungen elektrisch, elektromagnetisch, elektromotorisch, mit Hilfe mechanischer Systeme, mit Hilfe der Fluidik oder Pneumatik erzeugt werden oder durch den Einsatz piezoelektrischer/piezokeramischer Schwingungserzeuger bzw. Ultraschall-Schwingungserzeuger.

3. Verfahren nach Anspruch 1 oder 2, dadurch gekennzeichnet, daß die schwingungserzeugenden Systeme mit festen oder veränderlichen Frequenzen und mit festen oder veränderlichen Amplituden arbeiten.

4. Verfahren nach Anspruch 1, 2 oder 3, dadurch gekennzeichnet, daß die Ausbringungsmenge zusätzlich oder alternativ durch eine Veränderung der Düsenöffnung beeinflußt wird.

5. Vorrichtung zur Durchführung des Verfahrens nach Anspruch 1 bis 4, dadurch gekennzeichnet, daß sie einen Pulverbehälter mit einer kleinen Öffnung bzw. einer Düse umfaßt, die mittelbar oder unmittelbar mit einem Schwingungserzeuger gekoppelt sind, mit dem Schwingungen von der Frequenz der Eigenresonanz der Teilchen oder benachbarter Frequenzen auf die Teilchen übertragen werden können.

6. Vorrichtung nach Anspruch 5, dadurch gekennzeichnet, daß der Schwingungserzeuger mit einer Steuer-/Regeleinheit verbunden ist, die eine Veränderung der Frequenz, der Amplitude und gewünschtenfalls der Lage am Schwingungserzeuger entsprechend den Prozeßanforderungen ermöglicht.

7. Vorrichtung nach Anspruch 5 oder 6, dadurch gekennzeichnet, daß die Düse mit einem Verschlußmechanismus versehen ist, der mechanisch, elektrisch bzw. elektromagnetisch oder elektronisch betätigt wird.

8. Vorrichtung nach Anspruch 5, 6 oder 7, dadurch gekennzeichnet, daß eine Meßvorrichtung vorgesehen ist.

9. Vorrichtung nach Anspruch 8, dadurch gekennzeichnet, das die Entnahme aus der Meßvorrichtung nach Menge und Ausbringungsrichtung durch die Verwendung intermittierender Schieber oder rotierender Kreisscheiben entsprechend den Prozeßanforderungen erfolgt.

10. Vorrichtung nach Anspruch 5 bis 9, dadurch gekennzeichnet, daß das - schwingungserzeugende System im Vorratsbehälter installiert ist und durch Fremderregung oder direkte Energiezufuhr von außen arbeitet.

FIG.1.

FIG.2.

FIG.3.

FIG.4.

FIG.5.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-1 401 446 (F.S. CARVER INC.) * Seite 1, Zeilen 13-15; Seite 1, Zeile 77 - Seite 2, Zeile 15; Seite 2, Zeilen 58-75; Seite 3, Zeile 124 - Seite 4, Zeile 39; Figur 3 * | 1-3,6 | B 01 J 4/02 B 65 D 88/66 // A 61 M 15/00 |
| Y | | 9 | |
| X | FR-A-2 419 235 (COMPAGNIE INDUSTRIELLE DES TELECOMMUNICATIONS) * Seite 1, Zeilen 1-3,20-31; Seite 1, Zeile 38 - Seite 3, Zeile 14; Figuren 1,2 * | 1-3,5,7 ,10 | |
| X | US-A-4 583 660 (M.A. LA BARRE et al.) * Zusammenfassung; Figuren 1-3; Spalte 1, Zeile 59 - Spalte 2, Zeile 17; Spalte 2, Zeile 34 - Spalte 4, Zeile 48 * | 1-3,6 | |
| X | US-A-4 472 091 (J.W. CALLAHAN) * Zusammenfassung; Figuren 1-4; Spalte 2, Zeile 36 - Spalte 4, Zeile 66 * | 1-6,8 | RECHERCHIERTE SACHGEBIETE (Int. Cl.4) |
| Y | FR-A-1 303 147 (ETS. DE BONDUWE) * Seite 1, Spalte 1, Zeilen 1,2; Seite 1, Spalte 1, Absatz 4 - Spalte 2, Absatz 1; Seite 1, Spalte 2, Absatz 10 - Seite 2, Spalte 1, Absatz 7; Figuren 1,2 * | 9 | A 61 M B 01 J B 65 D |

Der vorliegende Recherchenbericht wurde für alle Patentansprüche erstellt

| Recherchenort | Abschlußdatum der Recherche | Prüfer |
|---|---|---|
| DEN HAAG | 08-06-1988 | SIEM T.D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
...........................................................................
& : Mitglied der gleichen Patentfamilie, übereinstimmendes Dokument

EPO FORM 1503 03.82 (P0403)